# EUROPEAN PATENT SPECIFICATION

(11) **EP 1 649 875 B1**
(45) Date of publication and mention of the grant of the patent: **12.10.2011**
(21) Application number: 04748282.3
(22) Date of filing: 02.08.2004
(51) Int. Cl.: A61L 27/38, A61L 27/12, A61L 27/56, C12N 5/07

(54) **METHOD OF CONSTRUCTING ARTIFICIAL JOINT**
VERFAHREN ZUR HERSTELLUNG EINES KÜNSTLICHEN GELENKS
PROCEDE DE FABRICATION D'UNE ARTICULATION ARTIFICIELLE

(30) Priority: 31.07.2003 JP 2003283703
(43) Date of publication of application: 26.04.2006
(73) Proprietor: Iwamoto, Yukihide, Fukuoka-shi, Fukuoka 8128582 (JP); Nakayama, Koichi, Fukuoka-shi, Fukuoka 8128582 (JP)
(72) Inventor: IWAMOTO, Yukihide Kyushu Uni., Dept of Orth. Surg., Higashi-ku, Fukoaka-shi, Fukuoka 812582 (JP); NAKAYAMA, Koichi Kyushu Uni., Dept. of Orth. Surg., Higashi-ku, Fukuoka-shi, Fukuoka 812582 (JP); TANAKA, Kazuhiro Kyushu Uni. Dept. of Orth. Surg., Higashi-ku, Fukuoka-shi, Fukuoka 812858 (JP); MATSUDA, Syuichi Kyushu Uni., Dept. of Orth. Surg., Higashi-ku, Fukuoka-shi, Fukuoka 812858 (JP)
(74) Representative: Woods, Geoffrey Corlett
(86) International application number: PCT/JP2004/011391
(87) International publication number: WO 2005/011765

(56) References cited:
- WO-A-2004/042038
- CA-A1- 2 254 350
- JP-A- 2002 078 484
- JP-A- 2002 537 896
- JP-A- 2003 038 635
- JP-A- 2003 111 831
- JP-A- 2003 304 866
- ANDERER U ET AL: "IN VITRO ENGINEERING OF HUMAN AUTOGENOUS CARTILAGE" JOURNAL OF BONE AND MINERAL RESEARCH, NEW YORK, NY, US, vol. 17, no. 8, August 2002 (2002-08), pages 1420-1429, XP009026956 ISSN: 0884-0431
- R.S. TUAN, G. BOLAND AND R. TULI: "Adult Mesenchymal Stem Cells and Cell-based Tissue Engineering" ARTHRITIS RESEARCH AND THERAPY, vol. 5, no. 1, 11 December 2002 (2002-12-11), pages 32-45, XP002458822
- JOHNSTONE BRIAN ET AL.: 'In vitro chondrogenesis of bone marrow-derived mesenchymal progenitor cells' EXPERIMENTAL CELL RESEARCH vol. 238, 1998, pages 265 - 272, XP000882426
- TOSHIHIRO IZUMI ET AL.: 'Chondrocyte transplantation for osteochondral defects with the use of suspentsion culture' CELL AND TISSUE BANKING vol. 1, 2000, pages 207 - 212, XP002971302

## Description

### TECHNICAL FIELD

The present invention relates to a method of preparing an artificial joint. Specifically, the present invention relates to a basic technology for repairing a cartilage layer throughout the relevant joint surface by loading a cell layer with an appropriate thickness to any curved surface.

### BACKGROUND ART

Cartilage is a connective tissue composed of chondrocytes and cartilage matrix, and joint bone is a tissue covered with cartilage of a sufficient thickness. Joints are supported by cartilage to allow smooth movement. However, once the cartilage is damaged or destroyed, it is extremely rare for such cartilage to be cured naturally. Therefore, in cases where the function of joints is greatly reduced because of osteoarthritis, articular rheumatism or the like, replacement of the joints with artificial joints composed of a metal, ceramics or polyethylene has been selected. This is a surgical method in which the damaged articular cartilage is excised and the resultant joint surface is covered with an artifact such as a metal, ceramics or polystyrene. This method is extensively used throughout the world, and it is still a major treatment method. The usefulness of this method has been confirmed for more than ten years with improvement of materials or designs.

However, materials such as metals, ceramics or polyethylene are foreign substances to the living body and do not have the ability to self-repair. Further, various problems resulting from materials, e.g., friction, corrosion fatigue, loosening in the interface between an artificial joint and bone, sinking, infection, etc. arise. Actually, there are a great number of cases where a re-operation becomes inevitable because artificial joints have been worn away and damaged.

Therefore, development of a new treatment method that will take over the current method using an artificial joint composed of an artifact as a material has been desired.

Recently, research of regeneration of tissues or organs based on ES cells or autologous cells has been studied vigorously. With respect to articular cartilage, technologies using various tissue engineering have been developed to treat partial defects. For example, a method has been developed in which autologous cells are transplanted at a site of defect to thereby induce secretion of regeneration factors from the surrounding healthy sites and expect the curing of the defect (Treatment of deep cartilage defects in the knee with autologous chondrocyte transplantation. N Engl J Med. 1994 Oct 6; 331(14):889-95). However, since this method merely fills up the site of defect, it is difficult to apply this method as a method for treating a wide range of joint destructions.

On the other hand, Vacanti et al. reported a study regarding regeneration of ear or finger defects (Transplantation of chondrocytes utilizing a polymer-cell construct to produce tissue-engineered cartilage in the shape of a human ear. Plast Reconstr Surg. 1997 Aug, 100(2):297-302). In this method, cells from a patient are adhered onto a carrier (made of polymer or the like) which has been formed in the shape of an ear or finger, then this carrier is transplanted subcutaneously into nude mice to thereby promote the production of extracellular matrices (such as collagen) using the nutrition supplied by the mice; and finally a shape of transplantation tissue is obtained. Nude mice are defective in the immune system. Therefore, no rejection occurs when cells from a patient are expanded in them and then re-transplanted into the patient; thus, nude mice are very useful.

However, since the size of mice has a limit, the size of tissues which can be prepared by this method depends on the size of mice. Besides, this method has problems from the viewpoint of religion or the prevention of cruelty to animals. Further, sense of rejection toward animals and the risk of infections represented by mad cow disease must always be taken into consideration. Although attempts have been made to use larger animals than mouse (e.g., pig) in regenerative medicine after making their immunological ability defective, solutions have not been found toward such problems as repulsion for the act of transplanting a tissue that once was in a heterologous animal body into a human body and the risk of unknown infections.

Another method has been disclosed in which a template of gel is prepared; cell suspension is adhered around the template; and then cells are adhered to a polymer or the like (Japanese Patent Publication No. 2003-144139). The purpose of this method is adhesion of cell suspension. Therefore, only membrane-like products are obtained by this method and it is difficult to prepare a tissue such as articular cartilage with a certain thickness by this method.

In order to solve the above problem, a method has been disclosed in which chondrocytes are adhered onto cotton-like scaffolds made of a degradable polymer to give a thickness to the part corresponding to articular cartilage. However, according to this method, the polymer remains in the articular cartilage part and changes into harmful substances during the process of degradation; thus, it is apprehended that this may give bad influences upon cells (Chondrogenesis in a cell-polymer-bioreactor system. Exp Cell Res. 1998 Apr 10; 240(1):58-65).

Another attempt has been made to regenerate cartilage by embedding cells in collagen. However, since the collagen is derived from animals such as bovine, this method seems to have a problem of affinity for the transplantation recipient bed (Transplantation of cartilage-like tissue made by tissue engineering in the treatment of cartilage defects of the knee. J Bone Joint Surg Br. 2002 May; 84(4):571-8).

CA-A-2,245,350 is concerned with a method for bioengineering cartilage.

### DISCLOSURE OF INVENTION

As described above, development of a new artificial joint that can take over the conventional artificial joint made of a metal, ceramics or polyethylene, is effective for a wide range of joint destructions, and is capable of wiping out the repulsion for using heterologous animals and the risk of unknown infections has been desired.

As a result of extensive and intensive researches toward the solution of the above problems, the present inventors have found that it is possible to form a cartilage of interest by adhering cell masses onto the surface of any carrier and culturing the cell masses under specific conditions. Thus, the present invention has been achieved.

Briefly, the present invention relates to a method of preparing a cartilage or an artificial joint surface, comprising adhering cell masses onto the surface of a carrier shaped into a desired form and having micropores and culturing *in vitro* or *ex vivo* the cell masses under conditions which induce differentiation of the cell masses into a cartilage tissue.

As the joint surface, a surface which is formed with the cells, matrices produced by the cells, or a combination thereof may be given.

In the method of the present invention, specific examples of the carrier include a carrier made of calcium triphosphate. As the cells, mesenchymal stem cells or chondrocytes may be given. The culture of the cells is performed, for example, *ex vivo* and in the presence of a growth factor (s).

### BRIEF DESCRIPTION OF DRAWINGS

Fig. 1 is diagrams showing a spherical joint surface consisting of cells alone.
Fig. 2 is a diagram showing a carrier designed based on three-dimensional data on the distal part of rabbit femur.
Fig. 3 is a diagram showing that cell masses have been adhered onto a carrier.
Fig. 4 is diagrams showing that additional cell masses have been adhered onto a carrier.
Fig. 5 is diagrams showing an overview of the distal joint surface of rabbit femur.

### BEST MODE FOR CARRYING OUT THE INVENTION

Hereinbelow, the present invention will be described in detail.

The present invention provides a method for preparing an autologous cell-derived cartilage or artificial joint surface which has a necessary and sufficient thickness of cell layer and is capable of loading to any curved surface. The method of the present invention is characterized by adhering cell masses onto the surface of a carrier shaped into a desired form and then culturing the cell masses under in vitro or ex vivo conditions which induce differentiation of the cell masses into a cartilage tissue. As a result, the cell masses are fused to each other along the shape of the carrier to thereby regenerate a joint surface, which functions as an artificial joint. Further, by adding a growth factor or a dynamic load, production of extracellular matrices is promoted, which results in *ex vivo* preparation of ideal articular cartilage even in terms of strength.

### 1. Cells

Cells to be cultured are undifferentiated cells such as stem cells (ES cells - not including ES cells obtainable by the destruction of human embryos, cord blood-derived cells, undifferentiated mesenchymal stem cells, etc) or differentiated cells therefrom. In particular, undifferentiated mesenchymal stem cells are preferable.

Since it is easy to induce differentiation of osteoblasts and chondrocytes from undifferentiated mesenchymal stem cells, such differentiation-induced cells (articular chondrocytes, osteocytes, etc) may also be used. Alternatively, adult mesenchymal stem cells may be used.

Further, stem cells may be cultured in a mixture with a plurality of types of cells (e.g., a combination of osteocytes and chondrocytes) at any site. For example, the cell layer may be combined the upper layer may consist of cell masses of chondrocytes alone with the lower layer may consist of osteocytes-derived cell masses and be two layers. In cases of the femur, cell masses of chondrocyte-type cells may be adhered onto the articular part; and cell masses of osteocytes-type cells may be adhered onto the bone part. Such mix culture is applicable to regeneration of the entire bone including the joint surface.

The above-described cells may be taken from experiment animals such as mouse, rabbit, rat, guinea pig, dog, pig, goat or bovine, or the bone marrow of patients by known techniques such as the Dexter method, the magnetic beads method, cell sorting, or the like.

Cells may be roughly classified into suspending cells and scaffold-dependent cells; blood system and immune system cells belong to the former, while cells of skin, bone and the like belong to the latter. Since cells of skin, bone and the like die when they are put in a state of suspension in culture broth, they proliferate by adhering onto plates made of glass or the like. Therefore, when cells are gathered at one place in Teflon^{™}, cells searching for a scaffold adhere to each other to thereby form a cell aggregate mass, i.e., spheroid. Further, when spheroids are adhered/fused to each other, a larger spheroid is formed.

As described in the 3rd edition of "Molecular Biology of the Cell", it is known that cells separated into single cells by enzyme treatment or the like aggregate spontaneously. It is known that this phenomenon is observed in cells of animals ranging from lower animals such as echinoid to mammals. This spontaneous aggregation is caused by an extracellular adhesion factor designated cell adhesion molecule (CAM) containing cadherin; and it is believed that a phenomenon almost similar to the mesenchymal stem cell aggregation occurring at the formation of four limbs during the early developmental stage of organisms is repeated in adult individuals (Gerisch, G Curr. Top. Dev. Biol. 14: 243-270, 1980; Hennings, H. Exp. Cell Res. 143: 127-142, 1983; Moscona, A.A.; Hausman, R.E. Biological and biochemical studies on embryonic cell-cell recognition. In Cell and Tissue Interactions, Society of General Physiologists Series (J.W. Lash, M.M. Burger, eds.), Vol. 32, pp. 173-185, New York: Raven Press, 1977; Roth, S.; Weston, J. Proc. Natl. Acad. Sci. USA 58: 974-980, 1967).

Still more recently, a report has been made which suggests that cadherin-mediated cell-cell adhesion functions as a switch to initiate the expression of collagen, etc. in the differentiation of mesenchymal stem cells into chondrocytes (Yoon YM, J Cell Biochem 2002; 87(3):342-59).

In the present invention, preferably, cells are allowed to form spheroids and then shaped into a specific form. In the present invention, the above-described mesenchymal cells or the like are monolayer-cultured and then transferred into a volatile or non-cell-adhesive round-bottomed multi-well or U-shaped well, followed by incubation. As a result, cells spontaneously aggregate, yielding cell masses (cell aggregate masses). The incubation time until the formation of cell masses is 6-48 hours, preferably 24-48 hours. In addition to the above-described method of preparation of cell masses, other methods may also be used. For example, the rotating culture method in which cell suspension is placed in a rotating solution; a method in which cell suspension is placed in test tubes and centrifuged for precipitation; the alginate beads method; or the like may be used. The above-described method of placing cell suspension in a volatile or non-cell-adhesive multi-well is preferable in terms of efficiency because uniform cell masses can be handled in a large quantity.

By the formation of the above-described spheroid, it is believed that the cells enter a quiescent state in the cell cycle and that their protein production increases. To allow cells to differentiate after induction into a quiescent state is expressed as "cells deviate from proliferation cycle and proceed to cell differentiation".

### 2. Culture of Cell Masses

Subsequently, the cell masses prepared as described above are adhered onto a carrier which has been shaped into a desired form, and then cultured. The number of cell masses is not particularly limited; any number may be selected. Further, if necessary, additional cell masses may be adhered after a certain number of cell masses have been adhered. The additional cell masses will fuse to the existing cell masses.

In the present invention, the carrier onto which cell masses are adhered may be designed in any form based on the structure of a joint of interest, i.e., a joint of human, rabbit, or the like. When a three-dimensional shape such as the ear, nose, etc. is to be obtained, a template may be designed in advance based on three-dimensional data to prepare the carrier.

The term "carrier" means a material which cell masses are capable of adhering onto, composes the whole or a part of an artificial joint, and functions as a support for cell masses. Preferably, this carrier is a biocompatible material which is absorbed and replaced with a self-tissue in the living body. Further, the surface of the carrier has micropores. As a specific example of such a carrier, a carrier made of calcium triphosphate may be given. Biodegradable polymers such as high molecular weight lactic acid polymers are also applicable.

With respect to those areas in the carrier which do not need adhesion of cell masses, it is possible to mask the areas with a volatile or non-cell-adhesive material.

Cell masses which have been adhered onto the carrier fuse to each other based on a mechanism almost equivalent to wound healing that organisms inherently have. As a result, they form a structure having the size and cell structure of interest.

Since the present invention aims at adhering cell masses to a carrier with a three-dimensional structure, it is necessary to make the height or thickness of the culture broth long. As a result, cells are present in the depth far from the surface of culture broth, which lowers the efficiency of gas exchange.

Then, in the present invention, the volume of culture broth was adjusted so that a part of cell masses touches the gas phase to some extent (to such an extent that will not dry the exposed part). Further, the culture broth was fed to cell masses through micropores of the carrier. The term "micropore" means a pore having a size that does not pass cell masses but passes the culture broth (diameter 10-500 µm). Micropores may be formed in the process of preparing of the carrier itself. Alternatively, it is possible to make micropores by sticking the carrier with thin needles.

Since the culture broth is capable of passing the micropores of the carrier freely, it is possible to supply the culture broth to cells constantly. Thus, cell masses are capable of undergoing differentiation, maturation and fusion/maturation among cell masses satisfactorily. It should be noted that the term "maturation" used here refers to increase in extracellular matrices produced by cells, such as various collagens and proteoglicans. Naturally, cells in the living body are often surrounded by abundant extracellular matrices.

The volume of culture broth is not particularly limited as long as cells are capable of differentiation/proliferation. However, at least 0.5 ml of culture broth in total volume is necessary per cell mass.

Specific examples of the culture broth which may be used for the culture include 10% FBS containing DMEM medium and serum-free DMEM high glucose medium.

The culture is performed under conditions which can induce the differentiation of cell masses into cartilage. The induction of the differentiation from cell masses to cartilage may be performed by culturing cells in the presence of a growth factor(s). Examples of growth factor(s) which may be used in the present invention include Bone Morphogenetic Protein (BMP), Fibroblast Growth Factor (FGF), Transforming Growth Factor-β (TGF-β), Insulin-like Growth Factor (IGF), Platelet Derived Growth Factor (PDGF) and Vascular Endothelial Growth Factor (VEGF).

The above-described factors are selected and added independently or in combination in appropriate amounts depending on the size to be achieved and the yields of extracellular matrices. For example, for differentiation of mesenchymal stem cells into chondrocytes, TGF-β is added. When BMP, FGF, IGF and the like are added further, a more collagen-rich cartilage-like tissue can be obtained.

It is known that when some stimulus is given to cells, generally the mRNA level increases and subsequently protein production increases. Paying attention to this fact, the inventors adjusted the degree of maturation of cells by varying culture conditions (culture period and other conditions). The "degree of maturation" means a degree which is suitable to transplant cell masses into defective pats of bones or joints. The time of transplantation may be examined the following: (1) cell masses are transplanted at the time point when mRNA has been increased; (2) cell masses are transplanted during the course of increase of proteins (extracellular matrices such as collagens); or (3) cell masses are transplanted at the time point when proteins have been produced sufficiently (i.e., maturation).

In cases of artificial joints, it is preferable to perform transplantation when proteins have been produced sufficiently. Preferably, the culture period is one month or more. For this purpose, it is important to use a carrier which does not change for a long period *in vitro.*

Generally, RNA production reaches its peak two weeks after the start of induction culture. Then, increase in collagen production is observed, followed by stabilization in 5 to 6 weeks.

Further, by adding various growth factors to cells, it is possible to adjust the amounts of extracellular matrices. It is also possible to increase extracellular matrices by adding mechanical stimuli (such as hydrostatic pressure or shearing elasticity), ultrasound, shock wave, or the like to cells. Therefore, it is possible to expedite the time period required for cells to reach the peak of RNA production by adding the above-described growth factors or mechanical stimuli such as hydrostatic pressure or ultrasound.

As a growth factor(s), BMP, FGF, TGF-β, IGF, PDGF, VEGF or the like as described above may also be used.

Thus, it is possible to adjust the degree of maturity freely by adjusting culture conditions or culture period.

Different from the conventional method in which regenerative tissues are temporarily transplanted subcutaneously into nude mice and matured there, the method of the present invention requires no animals. Therefore, desired forms of any size can be obtained without the limitation of the sizes of mouse bodies.

By using undifferentiated mesenchymal stem cells derived from patients themselves, it is possible to regenerate autologous cell-using tissues such as bones or cartilage.

Hereinbelow, the present invention will be described specifically with reference to the following Examples. However, the present invention is not limited to these Examples.

### EXAMPLE 1

The subject example simulates the head of femur in human hip joint.

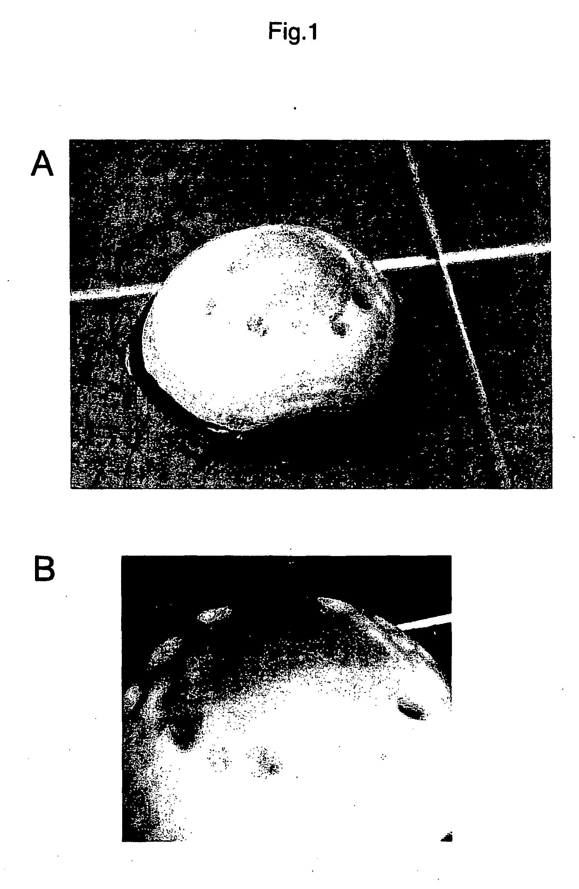

Human bone marrow-derived mesenchymal stem cells were monolayer-cultured. Finally, 1.0 x 10⁶ mesenchymal stem cells were obtained per 15 cm dish. The resultant cells were treated with trypsin, suspended and seeded in spheroid plates (Sumitomo Bakelite) at 1.0 x 10⁵ cells/plate. Then, the cells were cultured at 37°C under 5% CO₂ to thereby obtain 96 cell masses (with average diameter of 0.5 mm) per plate on the following day. These cell masses were adhered onto a calcium triphosphate carrier shaped into a semi-spherical form. Then, after addition of TGF-β at 0.01 µg/ml, the cells were cultured for 48 hours to thereby differentiate cell masses into cartilage. The cell masses adhered onto the curved surface fused to each other in 24 to 48 hours. Thus, a spherical joint surface consisting of cells alone was obtained (Fig. 1). In Fig. 1, panel B is an enlarged view of panel A.

### EXAMPLE 2

The subject example illustrates the preparation of distal joint surface of rabbit femur.

Sections of rabbit articular cartilage were treated with collagenase, and the resultant chondrocytes were monolayer-cultured. Finally, 1.0 x 10⁶ chondrocytes were obtained per 15 cm dish. The resultant cells were treated with trypsin, suspended and seeded in spheroid plates (Sumitomo Bakelite) at 1.0 x 10⁵ cells/plate. Then, the cells were cultured at 37°C under 5% CO₂ to thereby obtain 96 cell masses (with average diameter of 0.5 mm) per plate on the following day.

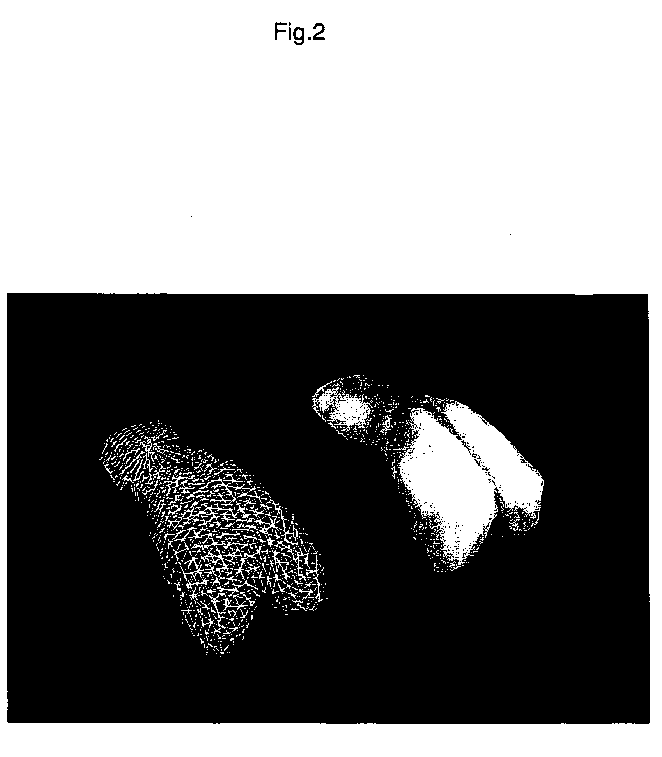

On the other hand, three-dimensional data were prepared in advance simulating the form of distal joint surface of rabbit femur (Fig. 2). Then, a carrier consisting of calcium triphosphate was prepared and shaped into this form.

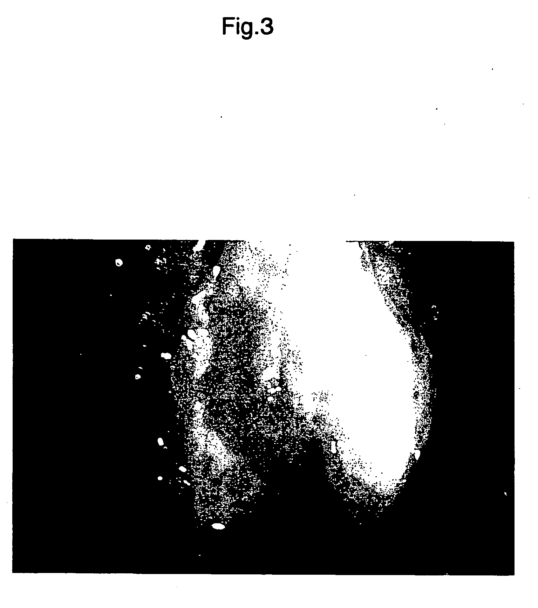

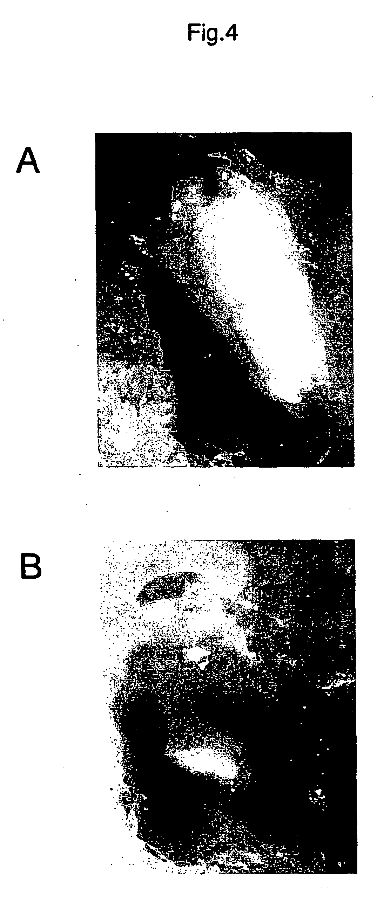

The cell masses prepared above were adhered onto the calcium triphosphate. The distal joint surface of femur made of calcium triphosphate is provided with micropores. Then, after addition of TGF-β at 0.01 µg/ml, the cells were cultured for 48 hours to thereby differentiate cell masses into cartilage. The cell masses adhered onto the curved surface fused to each other in 24 to 48 hours. Thus, a joint surface consisting of cells alone was obtained (Fig. 3). In Fig. 3, the part with a slightly brown color represents the area onto which cell masses were adhered. In the subsequent week (i.e., after 7 days), additional 320 cell masses were adhered onto the thus obtained joint surface (Fig. 3). As a result, the newly adhered cell masses fused to the already adhered cell masses and formed a smooth curved surface (Fig. 4A).

Further, additional 320 cell masses were adhered onto the above described joint surface. As a result, a joint of interest could be prepared as shown in Fig. 4B. In Fig. 4B, the part with a brown color is the area onto which cell masses were adhered. The white parts surrounding this area represent paraffin resin used for masking in order to prevent adhesion of cell masses onto unnecessary area.

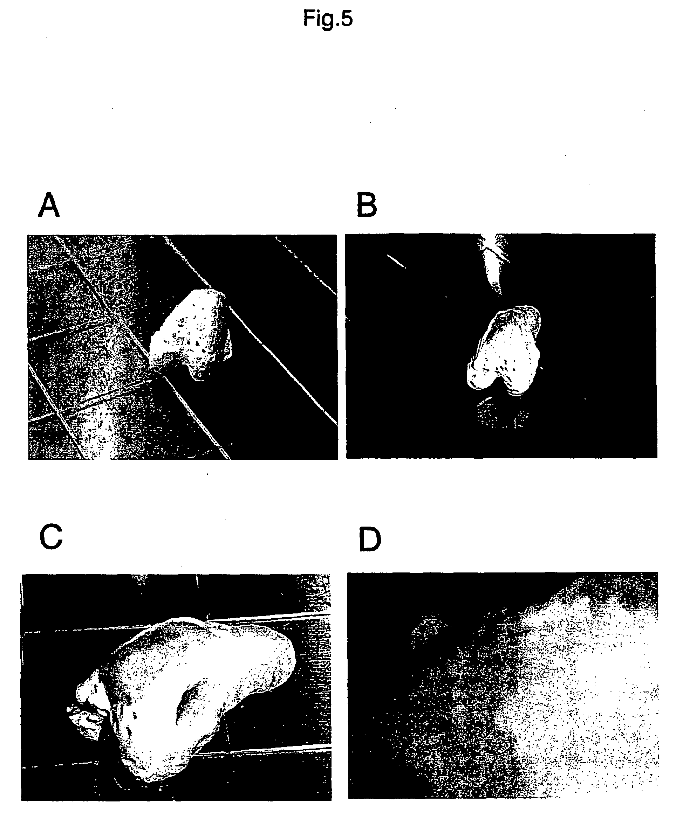

Fig. 5 shows an overview of the distal joint surface of rabbit femur.

Fig. 5A shows a carrier (before cell adhesion) consisting of calcium triphosphate shaped into the form of distal joint surface of rabbit femur that was prepared based on three-dimensional data input into a computer.

Fig. 5B and Fig. 5C show general views on the day after the adhesion of cell masses. It can be seen that a cell layer is only formed in the part corresponding to cartilage. Fig. 5D is an enlarged view of the area onto which cell masses were adhered (Fig. 5B and 5C). Fusion has proceeded to such an extent that the border of cell masses can be somewhat recognized.

### INDUSTRIAL APPLICABILTTY

According to the present invention, a method of preparing cartilage and artificial joints is provided. The technology provided by the present invention produces a large quantity of cell aggregate masses and fuses them to each other, to thereby enable preparation of an articular cartilage layer with autologous cells alone, without necessity of using carriers such as polymer. Further, since the method of the present invention does not require transplantation into animals to mature joints, it has become possible to prepare a cartilage layer of any form without mediation of heterologous animals.

## Claims

1. A method of preparing a cartilage or an artificial joint surface, comprising adhering cell masses onto the surface of a carrier shaped into a desired form and having micropores and culturing *in vitro* or *ex vivo* the cell masses under conditions which induce differentiation of the cell masses into a cartilage tissue.

2. A method according to claim 1, wherein the method is for preparing an artificial joint surface and the carrier is shaped into the form of a desired joint surface.

3. The method according to claim 1 or 2, wherein the cell masses are cultured to form a joint surface and the joint surface is formed with the cells, matrices produced by the cells or a combination thereof.

4. The method according to any one of claims 1-3, wherein the cells are mesenchymal stem cells or chondrocytes.

5. The method according to any one of claims 1-4, wherein the culture is performed *ex vivo* and in the presence of a growth factor(s).

## Patentansprüche

1. Verfahren zur Herstellung eines Knorpels oder einer künstlichen Gelenkoberfläche, umfassend das Anhaften von Zellmassen auf der Oberfläche eines Trägers, gestaltet in einer gewünschten Form und mit Mikroporen, und in-vitro- oder ex-vivo-Kultivieren der Zellmassen unter Bedingungen, die die Differenzierung der Zellmassen in ein Knorpelgewebe induzieren.

2. Verfahren gemäß Anspruch 1, wobei das Verfahren zur Herstellung einer künstlichen Gelenksoberfläche ist und der Träger in der Form einer gewünschten Gelenksoberfläche gestaltet ist.

3. Verfahren gemäß Anspruch 1 oder 2, wobei die Zellmassen kultviert werden, um eine Gelenksoberfläche zu bilden und die Gelenksoberfläche mit den Zellen, Matrizen, produziert durch die Zellen, oder einer Kombination davon gebildet wird.

4. Verfahren gemäß einem der Ansprüche 1 bis 3, wobei die Zellen mesenchymale Stammzellen oder Chondrozyten sind.

5. Verfahren gemäß einem der Ansprüche 1 bis 4, wobei die Kultur ex-vivo und in der Anwesenheit von Wachstumsfaktor(en) durchgeführt wird.

## Revendications

1. Procédé de préparation d'un cartilage ou d'une surface d'une articulation artificielle, comprenant le fait de coller des masses cellulaires sur la surface d'un support ayant une forme souhaitée et comportant des micropores et le fait de cultiver in vitro ou ex vivo les masses cellulaires dans des conditions permettant d'amorcer la différenciation des masses cellulaires pour donner un tissu cartilagineux.

2. Procédé selon la revendication 1, dans lequel le procédé sert à préparer une surface d'une articulation artificielle et le support a la forme d'une surface d'articulation souhaitée.

3. Procédé selon la revendication 1 ou 2, dans lequel les masses cellulaires sont cultivées pour former une surface d'une articulation et la surface de l'articulation est formée avec les cellules, les matrices produites par les cellules, ou une combinaison des deux.

4. Procédé selon l'une quelconque des revendications 1-3, dans lequel les cellules sont des cellules souches mésenchymateuses ou des chondrocytes.

5. Procédé selon l'une quelconque des revendications 1-4, dans lequel la culture est réalisée ex vivo et en présence d'un ou plusieurs facteur(s) de croissance.
